# EUROPEAN PATENT APPLICATION

(11) **EP 4 726 647 A1**
(43) Date of publication of application: **15.04.2026**
(21) Application number: 24205888.1
(22) Date of filing: 10.10.2024
(51) Int. Cl.: G06T 7/00

(54) **METHODS AND SYSTEMS FOR TEMPORAL INTERPOLATION**

(71) Applicant: Siemens Healthineers AG, 91301 Forchheim (DE); Friedrich-Alexander-Universität Erlangen-Nürnberg, 91054 Erlangen (DE)
(72) Inventor: Maul, Noah, 91052 Erlangen (DE); Birkhold, Annette, 70193 Stuttgart (DE)
(74) Representative: Siemens Healthineers Patent Attorneys

(57) **Abstract**

The invention relates to systems comprising a device, in particular a digital subtraction angiography (DSA) device, and a method of temporal interpolation based on data measured by the device. The method comprises generating training data for a neural network; training the neural network using the generated training data; measuring a series of data frames on a sample; interpolating one or more data frames using the trained neural network based on a plurality of data frames of the measured series of data frames to increase a temporal resolution of the measured series of data frames and/or to extrapolate the measured series of data frames.

In DSA, the systems and methods enable increased accuracy of estimations of, for instance, a bolus arrival time (BAT) enabling, for instance, improved use of contrast agents (CAs) and/or improved diagnosis of circulatory issues while reducing the risk of unnecessary exposure to radiation. More generally, a time required for performing measurements may be reduced, thereby reducing costs and/or increasing the lifetime of the measurement device.

## Description

### FIELD

The present invention relates to methods and systems for temporal interpolation, in particular for temporal interpolation in digital subtraction angiography (DSA).

### BACKGROUND

DSA is an imaging technique which may for instance be used to visualize vessels and the dynamics of liquids flowing therein. In medical applications, DSA typically involves injecting a contrast agent (CA) into the bloodstream of a patient, then capturing X-ray images before and after the CA is introduced and/or while the CA is flowing in constant or varying concentrations. By subtracting a pre-contrast image from a contrasted image, DSA may eliminate surrounding tissues and may lead to a clear view of the blood vessels and/or the dynamics of the blood flowing therein.

In medical applications, DSA is commonly used for diagnosing conditions like aneurysms, stenosis, or vascular malformations. DSA may provide high-resolution images while being minimally invasive, making it valuable in both diagnostic and interventional radiology. However, DSA may involve the risks of the exposure to ionizing radiation, in particular when DSA is used to measure and/or estimate hemodynamic quantities involving high velocities of blood in blood vessels and thus requiring high measurement frequencies to achieve a required temporal resolution.

Bolus arrival time (BAT) may be mentioned as an example of a hemodynamic quantity which may require a high frequency of DSA measurements. BAT generally refers to the time it takes for a CA, after injection, to reach a target region, each a specific region in a blood vessels. BAT is a clinically relevant quantity for several reasons. Precise knowledge of the BAT enables precise synchronization between the arrival of the CA at the region of interest and the timing of the DSA measurement for that specific region. Knowing the BAT ensures that measurements are taken when the contrast is fully in the vessels, providing clear, accurate images for diagnosis. Knowledge on the BAT allows also for an assessment of the dynamics of the blood flow in the vessels. Delayed arrival may, for instance, suggest vascular abnormalities such as stenosis, occlusion, or reduced perfusion, which helps in diagnosing of circulatory issues. Moreover, knowledge on the BAT allows for optimizing the dosage of the CA to reduce the risk of overuse or unnecessary radiation exposure.

Estimations of the BAT may be challenging due to geometries and/or complex networks of blood vessels and differing velocities of blood therein. Between two acquired images, a CA may have travelled over a relatively large distance and in different concentrations, leading to inaccurate BAT estimations and error propagation for secondary quantities derived from the BAT, including medical diagnosis.

Against this background, the problem addressed by the present invention is that of providing method and system in DSA enabling increased accuracy of estimations of the BAT to enable improved use of CAs and/or improve diagnosis of circulatory issues while reducing the risk of unnecessary radiation exposure. More generally, a time required for performing measurements may be reduced, thereby reducing costs and/or increasing the lifetime of the measurement device.

### SUMMARY

These problems are solved according to the invention by a method and systems for temporal interpolation in, e.g., digital subtraction angiography according to independent claims 1, 9, and 10.

Advantageous configurations and developments emerge from the dependent claims and from the description with reference to the figures of the drawings.

According to a first aspect, the invention relates to a method of temporal interpolation comprising: generating training data for a neural network; training the neural network using the generated training data; measuring a series of data frames on a sample; interpolating one or more data frames using the trained neural network based on a plurality of data frames of the measured series of data frames to increase a temporal resolution of the measured series of data frames and/or to extrapolate the measured series of data frames.

According to a further development, the method further comprises deriving hemodynamic quantities based on the series of measured data frames and the one or more interpolated data frames; and/or presenting a series of data frames including the measured series of data frames and the one or more interpolated data frames.

According to a further development, generating training data comprises reconstructing vessels in three dimensions or reconstructing vessels in three dimensions at a plurality of points in time based on a model and/or clinical data obtained from one or more samples.

According to a further development, generating training data further comprises extracting boundary conditions for hemodynamic quantities based on the reconstruction; and/or performing fluid dynamics simulations in the reconstruction to derive synthetic hemodynamic quantities and/or one or more synthetic data frames.

According to a further development, generating training data further comprises generating the training data from clinical data obtained from a plurality of devices, or generating the training data only from data recorded from a single patient.

According to a further development, training the neural network using the generated training data further comprises deriving hemodynamic quantities using the neural network, comparing the deriving hemodynamic quantities with the extracted boundary conditions for hemodynamic quantities, and adjusting the neural network to reduce a deviation; and/or interpolating one or more data frames using the neural network, comparing the interpolated one or more data frames with the one or more synthetic data frames, and adjusting the neural network to reduce a deviation.

According to a further development, a temporal resolution of the presented series of data frames has a frequency in the range between 30 and 200Hz and is greater than a temporal resolution of the measured series of data frames having a frequency in the range between 1 and 30 Hz.

According to a further development, the interpolated one or more data frames comprise at least one interpolated data frame for a time instance before or after each of the time instances associated with the measured data frames of the measured series of data frames to extrapolate the measured series of data frames, wherein the time instances preferably refer to the time after injection of a contrast agent into a vessel.

According to a second aspect, the invention relates to a non-volatile computer-readable storage medium comprising instructions stored thereon, which, when executed by a processor, cause the processor to perform the method described above.

According to a third aspect, the invention relates to a system comprising a processor; means configured to generate training data for a neural network; means configured to train the neural network using the generated training data; means configured to measure a series of data frames on a sample; means configured to interpolate one or more data frames using the trained neural network based on a plurality of data frames of the measured series of data frames to increase a temporal resolution of the measured series of data frames and/or to extrapolate the measured series of data frames.

Where appropriate, the above-mentioned configurations, developments and implementations can be combined with each other as desired, as far as this is reasonable. Further possible configurations, developments and implementations of the invention also include combinations, which are not explicitly mentioned, of features of the invention which have been described previously or are described in the following with reference to the embodiments. In particular, in this case, a person skilled in the art will also add individual aspects as improvements or supplements to the basic form of the present invention.

### DESCRIPTION OF THE DRAWINGS

The present invention is described in greater detail in the following on the basis of the embodiments shown in the schematic figures of the drawings, in which:

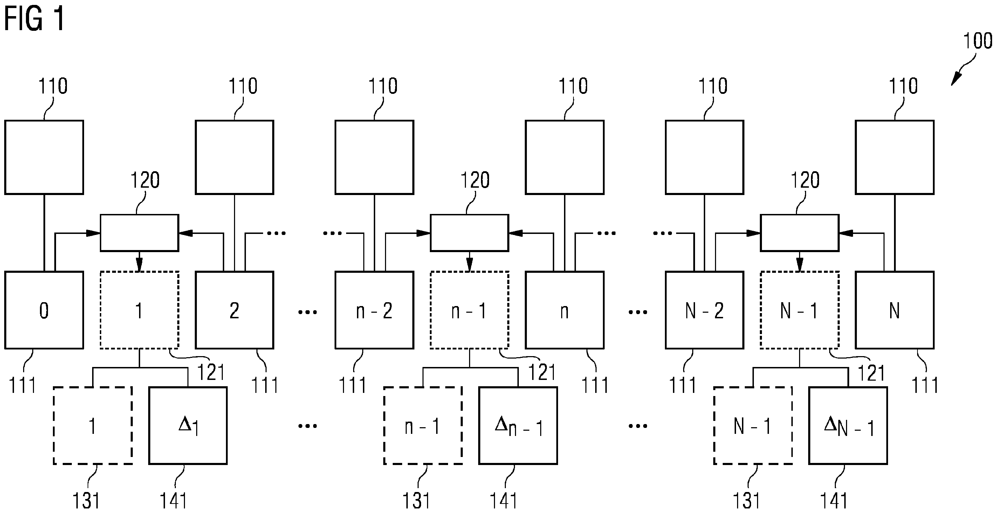

- Fig. 1:: illustrates a scheme of an exemplary system providing a series of data frames recorded at different instances in time and a corresponding method of temporal interpolation that is based on the series of data frames to obtain interpolated data frames including a comparison of the interpolated data frames with training or reference data frames obtained from one or more sources to enable improving the method of temporal interpolation based on the series of data frames and/or the reference data frames obtained; and

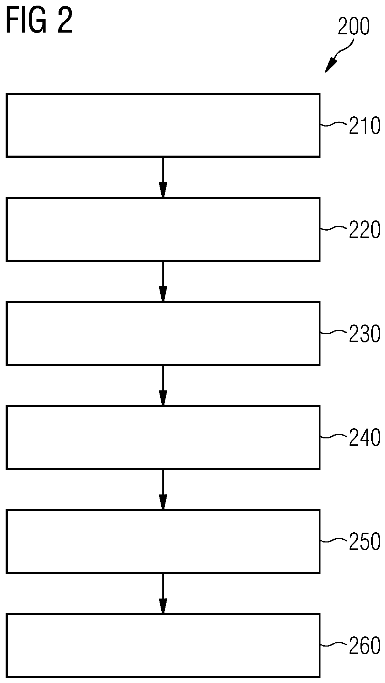

- Fig. 2:: illustrates exemplary steps of a method of temporal interpolation using a series of data frames to obtain interpolated data frames including comparing the interpolated data frames with training or reference data frames obtained from one or more sources to enable improving the method of temporal interpolation based on the series of data frames and/or the training or reference data frames obtained.

The appended drawings are intended to provide further understanding of the embodiments of the invention. They illustrate embodiments and, in conjunction with the description, help to explain principles and concepts of the invention. Other embodiments and many of the advantages mentioned become apparent in view of the drawings. The elements in the drawings are not necessarily shown to scale.

In the drawings, like, functionally equivalent and identically operating elements, features and components are provided with like reference signs in each case, unless stated otherwise.

Fig. 1 illustrates a scheme of an exemplary system 100 providing a series of data frames 111, recorded by, *e.g.,* a device 110 such as a DSA device, at different instances in time. The different instances in time in the series are, with loss of generality (wlog), enumerated by 0, 2, up to N where N denotes is a finite integer. The series of data frames 111 may be obtained from different sources, for instance from different DSA devices, and/or from one or more databases collecting data recorded by a plurality DSA devices. Device 110 may thus represent one or more different devices, in particular different DSA devices.

The series of data frames 111 may be equally spaced in time with a measurement frequency in a range between 5 and 1000 Hz, preferably a range between 10 and 100 Hz, more preferably a range between 20 and 50 Hz, even more preferably a range between 25 and 40Hz. A frequency of 30 Herz may be mentioned as an example for an ideal trade-off between the advantages of a temporal resolution enabling improved diagnosis and the risks due to the associated exposure to radiation. Equal spacing in time for the series of data frames 111 is, however, not required. The spacing in time of directly successive frames 111 may also vary for a number of pairs or each pair of directly successive data frames 111.

The series of data frames 111 may also comprise a plurality of series of data frames as obtained from a plurality of measurement occasions at a single device. Such series of data frames may, for instance, be collected for a single patient at a plurality of meetings at the measurement site where the single device is located. Each series may have a measurement frequency, wherein the measurement frequencies of different series of data frames may be equal or may be the same. Different series of data frames may also be relatively shifted in time with respect to the time of injection of the contrast agent. In such cases the measurement frequencies of the different series of data frames may be the same. In an example, training data for training a neural network 120 may may be obtained from several patients. Different inflow/outflow boundary conditions or injection parameters can be simulated for one or more patients to enlarge the dataset.

A neural network 120 may be used to interpolate a data frame 121 at a time instance at or between data frames 111 of the series recorded by the device 100. The neural network 120 may for instance be used to interpolate the data frames 121 at the instances in time enumerated with the enumerator 1, n-1, or N-1, as shown in Fig. 1, to increase the temporal resolution of the series of data frames. The instances in time for the interpolated data frames 121 may be selected be in the range between the time instances 0 and 2, n and n-2, or N-2 and N, respectively.

The instances in time for the interpolated data frames 121 may each have the same or different distances in time with respect to a directly preceding and with respect to a directly succeeding data frame 111. The neural network 120 may also use a different plurality of data frames 111 and/or further data frames 111 of one or more series of data frames to interpolate a data frame 121 at some time instance in or next to time range, e.g., 0 to N in Fig. 1, of the series.

Interpolating data frames in series of data frames may increase the number of data frames and thus lead to an increased number of frames per time interval. In other words, a measurement frequency for a series of data frames may be increased using interpolation. The resulting frequency may enable an increased accuracy of hemodynamic quantity computation based on the series of data frames including both data frames 111 from measurement on real samples and interpolated data frames 121 obtained from, e.g., the above-mentioned neural network 120 used for interpolation.

Training data for training the neural network 120 may be generated in different ways which may or may not be combined depending on circumstances and/or applications. Training data may, for instance, be based on clinical data obtained from DSA measurements performed on one or more patients. The DSA measurements may comprise information on the dynamics of the CA in the patients vessels. Said information may be used to reconstruct the patients vessels in three dimensions (3D) possibly including time in a fourth dimension (4D). The reconstructed vessels may, for instance, be made available in terms of time-dependent or time-grammatical termdent vascular volume meshes. These information and/or alternative forms thereof may be used to extract boundary conditions for the interpolated data frames for training.

Alternatively or in addition, for training, secondary information such as a specific diagnosis may be derived from vascular volume meshes or clinical data in general to extract boundary conditions for secondary information that is derived using information comprised in interpolated data frames. Sampling boundary conditions from the literature may also be used for training, at least in terms of secondary information derived from the available data.

Training data may also be synthesized trough simulations using, *e.g*., a 3D or 4D reconstruction of the shape of vessels and/or computational methods for simulation the fluid dynamics in reconstructed or modelled vessels. Such simulations may be used to compute CA filling states between measured data frames. The computed CA filling states may be compared with the filling states according to an interpolation. Training may then be performed by minimizing the deviation.

Computation of synthetic data frames through simulations enables the provision of series of data frames at essentially arbitrary temporal resolution.

Directly measured training data, training data derived therefrom and/or synthesized training data may be specifically generated for a single patient using only data collected from the single patient and/or synthesized data obtained from simulations using, *e.g*., a 3D or 4D reconstruction of the single patients vessels.

As illustrated in Fig. 1, training may be performed by comparing an interpolated data frame 121 with a training or reference data frame 131 and minimizing the corresponding deviation 141. More generally, alternatively or in addition, training may be performed by comparing information derived from the interpolated data frame 121 with corresponding training data derived as described above and minimizing the corresponding deviation 141.

In the non-limiting example shown in Fig. 1, two subsequent data frames, for example frame 121 at time instance 0 and frame 121 at time instance 2, are used for generating an interpolated data frame 121 at time instance 1. The interpolated data frame 121 at time instance 1 is then compared with a training data frame 131 for time instance 1 to minimize the deviation 141 for time instance 1. The same is shown for subsequent pairs of data frames. Alternative implementations are also possible. The example shown in Fig. 1 is compatible with each of the above-mentioned modifications. In particular a longer sequence of data frames including a plurality of measured data frames and/or hemodynamic or morphological information may be used for generating the interpolated data frame.

A temporal resolution of a series of data frames may not be limited to some specific frequency. The temporal resolution of a measured series of data frames, and/or one or more interpolated data frames, and/or one or more presented data frames (*cf*. presenting 260 in method 200 described below), and/or any other series of data frames described herein may have any frequency possible within the realm of physical laws.

Fig. 2 illustrates exemplary steps of a method 200 of temporal interpolation using a series of data frames to obtain interpolated data frames including comparing the interpolated data frames with training or reference data frames obtained from one or more sources to enable improving the method of temporal interpolation based on the series of data frames and/or the training or reference data frames obtained.

The method 200 comprises generating 210 training data for a neural network 120.

The training data may comprise information derived from clinical data. Deriving the information may comprise obtaining clinical data frames acquired using a DSA device. Deriving the information may further comprise reconstructing vessels in three dimensions. Reconstructing vessels in three dimensions may further comprise reconstructing vessels at a plurality of different points in time. Deriving the information may further comprise extracting boundary conditions for hemodynamic quantities for comparison with hemodynamic quantities derived based on information derived from interpolated data frames generated by the neural network

The training data may comprise information derived from synthetic data. Deriving the information may comprise obtaining synthetic data frames using simulation of fluid dynamics in vessels. The vessels may be reconstructed vessels in three or four dimensions. Deriving the information may further comprise extracting boundary conditions for hemodynamic quantities for comparison with hemodynamic quantities derived based on information derived from interpolated data frames generated by the neural network.

The method 200 further comprises training 220 the neural network 120 using the generated training data.

Training 220 the neural network may comprise generating interpolated data, in particular an interpolated date frame 121 based on a plurality of data frames 111 measured by one or more DSA devices. The plurality of data frames may comprise a pair of successive data frames 111, in particular a pair of directly successive data frames 111, such as, with reference to Fig. 1 and wlog, data frame 111 at time instance 0 and data frame 111 a time instance 2. Training 220 the neural network may further comprise deriving secondary information based at least in part on the interpolated data frame 121. Training 220 the neural network may further comprise comparing the interpolated data with the generated training data, in particular simulated data frames 121. Alternatively or in addition, training 220 the neural network may further comprise comparing the derived secondary information with secondary information obtained from clinical and/or synthetical data, in particular from simulations.

The method 200 further comprises measuring 230 a series of data frames 111. The measuring 230 may be performed on one or more samples, such as a patient for which an image of blood vessels is to be obtained in temporal resolution for, *e.g*., medical diagnosis. As described above in relation to Fig. 1, the series of data frames 111 may be obtained from different sources, for instance from different DSA devices, and/or from one or more databases collecting data recorded by a plurality DSA devices. The series of data frames 111 may be obtained for a single patient from one or more DSA devices.

The method 200 further comprises interpolating 240 one or more data frames 121 using the trained neural network 120 based on a plurality of data frames 111 of the series of data frames 111. Interpolation may be performed to increase the temporal resolution of the series of measured data frames 111.

The method 200 may further comprise deriving 250 hemodynamic quantities based on the series of measured data frames 111 and the one or more interpolated data frames 121.

Alternatively or in addition to the deriving 250, the method 200 may comprise presenting (260) a series of data frames including the series of data frames (111) and one or more interpolated data frames (112). The presenting (260) may be performed on a display, such as a display of the DSA device 110. Transmission to a display of a mobile device may also be performed, alternatively or in addition.

The method 200 may be implemented in computer executable instructions. The computer executable instructions may be stored in a non-volatile computer readable memory. The instructions, when executed by a computer, comprised in, *e.g*., the DSA device 110, are configured to perform the method 200. The method 200 and/or the neural network 120 may be implemented in the computer or separately, external to the DSA device 110, for example.

Although the present invention has been described in the above by way of embodiments, it is not limited thereto, but rather can be modified in a wide range of ways. In particular, the invention can be changed or modified in various ways without deviating from the core of the invention. Independent of the grammatical term usage, individuals with male, female or other gender identities are included within the term.

## Claims

1. Method (200) of temporal interpolation comprising:
generating (210) training data for a neural network (120);
training (220) the neural network (120) using the generated training data;
measuring (230) a series of data frames (111) on a sample;
interpolating (240) one or more data frames (121) using the trained neural network (120) based on a plurality of data frames (111) of the measured series of data frames (111) to increase a temporal resolution of the measured series of data frames (111) and/or to extrapolate the measured series of data frames (111).

2. The method (200) of claim 1 further comprising:
deriving (250) hemodynamic quantities based on the series of measured data frames (111) and the one or more interpolated data frames (121); and/or
presenting (260) a series of data frames including the measured series of data frames (111) and the one or more interpolated data frames (112).

3. The method (200) of any one of claims 1 to 2 wherein generating (210) training data comprises:
reconstructing vessels in three dimensions or reconstructing vessels in three dimensions at a plurality of points in time based on a model and/or clinical data obtained from one or more samples.

4. The method (200) of claim 3, wherein generating (210) training data further comprises:
extracting boundary conditions for hemodynamic quantities based on the reconstruction; and/or
performing fluid dynamics simulations in the reconstruction to derive synthetic hemodynamic quantities and/or one or more synthetic data frames.

5. The method (200) of any one of claims 3 to 4, wherein generating (210) training data further comprises generating the training data from clinical data obtained from a plurality of devices, or generating the training data only from data recorded from a single patient.

6. The method (200) of claim 4 or 5 wherein training (220) the neural network (120) using the generated training data further comprises:
deriving hemodynamic quantities using the neural network (120), comparing the deriving hemodynamic quantities with the extracted boundary conditions for hemodynamic quantities, and adjusting the neural network (120) to reduce a deviation; and/or
interpolating one or more data frames using the neural network (120), comparing the interpolated one or more data frames with the one or more synthetic data frames, and adjusting the neural network (120) to reduce a deviation.

7. The method (200) of claim 1, wherein a temporal resolution of the presented series of data frames has a frequency in the range between 30 and 200Hz and is greater than a temporal resolution of the measured series of data frames (111) having a frequency in the range between 1 and 30 Hz.

8. The method (200) of claim 1, wherein the interpolated one or more data frames (121) comprise at least one interpolated data frame (121) for a time instance before or after each of the time instances associated with the measured data frames (111) of the measured series of data frames (111) to extrapolate the measured series of data frames (111), wherein the time instances preferably refer to the time after injection of a contrast agent into a vessel.

9. Non-volatile computer-readable storage medium comprising instructions stored thereon, which, when executed by a processor, cause the processor to perform the method according to any one of claims 1 to 8.

10. System (100) comprising:
a processor;
means configured to generate (210) training data for a neural network (120);
means configured to train (220) the neural network (120) using the generated training data;
means configured to measure (230) a series of data frames (111) on a sample;
means configured to interpolate (240) one or more data frames (121) using the trained neural network (120) based on a plurality of data frames (111) of the measured series of data frames (111) to increase a temporal resolution of the measured series of data frames (111) and/or to extrapolate the measured series of data frames (111).
